# EUROPEAN PATENT APPLICATION

(11) **EP 4 458 354 A1**
(43) Date of publication of application: **06.11.2024**
(21) Application number: 22916827.3
(22) Date of filing: 30.12.2022
(51) Int. Cl.: A61K 31/40, A61K 31/704, A61K 31/337, A61K 45/06, A61P 35/00

(54) **PHARMACEUTICAL COMPOSITION FOR PREVENTING OR TREATING TRIPLE-NEGATIVE BREAST CANCER**

(30) Priority: 30.12.2021 KR 20210192406
(71) Applicant: Daewoong Pharmaceutical Co., Ltd., Hwaseong-si, Gyeonggi-do 18623 (KR)
(72) Inventor: KWON, Ahreum, Seoul 06170 (KR); LIM, Kwon Jo, Seoul 06170 (KR); KIM, Ji Duck, Seoul 06170 (KR); PARK, Joon Seok, Seoul 06170 (KR)
(74) Representative: Germain Maureau
(86) International application number: PCT/KR2022/021693
(87) International publication number: WO 2023/128686

(57) **Abstract**

The present invention provides a pharmaceutical composition comprising a compound represented by Chemical Formula 1 described in the present specification, or a pharmaceutically acceptable salt thereof, and a chemotherapeutic agent. The pharmaceutical composition can be usefully used for preventing or treating triple-negative breast cancer.

## Description

### [TECHNICAL FIELD]

The present invention relates to a pharmaceutical composition that can be usefully used for preventing or treating triple-negative breast cancer.

### [BACKGROUND]

Cancer is a disease whose incidence is increasing every year due to lifestyle and environmental changes, aging of the population, etc., and ranks as the top causes of death in Korea. In order to conquer cancer, therapeutic agents have evolved into first-generation chemical anticancer agents, second-generation targeted anticancer agents, and third-generation immuno-anticancer agents, which allowed a wide selection range of therapeutic agents, but the problem of therapeutic efficacy inhibition due to anticancer agent resistance has been steadily raised. In particular, therapeutic methods using a chemical anticancer agent such as Doxorubicin are still known to be the preferred approach in clinical cancer treatment (breast, bladder, ovarian, lung, stomach cancer, etc.), but the efficacy of anticancer agents is reduced due to the development of endogenous or acquired multidrug resistance in cancer cells.

Triple-negative breast cancer is a breast cancer in which Estrogen Receptor (ER), Progesterone Receptor (PR), and Human Epidermal Growth Factor Receptor 2 (HER2) are all negative, and therapeutic agents other than existing anticancer chemotherapy are limited. Further, triple-negative breast cancer patients have a high recurrence rate, and thus are in need of new therapeutic methods. In addition, the problem of therapeutic efficacy inhibition due to anticancer agent resistance is steady being raised. For triple-negative breast cancer patients for whom hormonal therapy for the treatment of breast cancer is not applicable, cytotoxic anticancer agent monotherapy is applied, or combination therapy with other cytotoxic anticancer agents or with targeted therapeutic agents is applied, taking into account their clinical status and biological characteristics. Anthracyclines including Doxorubicin, and taxanes such as docetaxel and paclitaxel can be regarded as representative drugs that have been widely used as a single drug or in combination with other drugs to date as the most effective cytotoxic anticancer agents, and is currently a first therapeutic drug for triple-negative breast cancer.

Various combination therapies have been proposed to overcome anticancer agent resistance and effectively treat tumors with high genetic heterogeneity. This approach could be used to design therapeutic methods for cancers having high lethal rates, such as acute lymphoblastic leukemia and Hodgkin lymphoma. Recently, it has been reported that among the chemoanticancer treatment regimens for triple-negative breast cancer patients, when omeprazole or Esomeprazole, which is a proton pump inhibitor (PPI) family drug, is taken before AC-T (adriamycin, cyclophosphamide, taxane) treatment, it exhibits a significant improving effect in pathologic complete response (pCR). In this way, combination therapy using existing drugs with guaranteed safety is continuously being researched.

On the other hand, Korean Patent No. 10-1613245 discloses that Fexuprazan is a 4-methoxy pyrrole derivative having proton pump inhibitory activity. Based on its proton pump inhibitory activity, it is known to have effects such as stomach damage inhibition activity, defensive factor enhancing effect, and excellent Helicobacter pylori (H. pylori) eradication activity.

Therefore, the present inventors conducted intensive research, and found that Fexuprazan, when used in combination with a specific chemotherapeutic agent, showed excellent effects in preventing or treating triple-negative breast cancer, thereby completing the present invention.

### [DETAILED DESCRIPTION OF THE INVENTION]

### [Technical Problem]

It is an object of the present invention to provide a pharmaceutical composition that can be usefully used for preventing or treating triple-negative breast cancer.

### [Technical Solution]

In order to achieve the above object, according to the present invention, there is provided a pharmaceutical composition for preventing or treating triple-negative breast cancer comprising: i) a compound represented by the following Chemical Formula 1, or a pharmaceutically acceptable salt thereof, and ii) a chemotherapeutic agent:

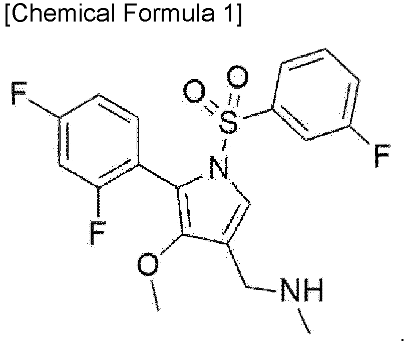

The compound represented by Chemical Formula 1 is a compound named 'Fexuprazan', whose IUPAC name is 1-(5-(2,4-difluorophenyl)-1-((3-fluorophenyl)sulfonyl)-4-methoxy-1H-pyrrol-3-yl)-N-methylmethanamine, and is a compound described in Korean Patent No. 10-1613245.

The above compound is an active ingredient that exhibits pharmacological effects of the pharmaceutical composition according to the present invention, and has excellent anti-ulcer activity (i.e., proton pump inhibitory activity, etc.), Helicobacter pylori (H. pylori) eradication activity and GPCR inhibition action, and thus is useful in the prevention and treatment of gastrointestinal track ulcer, gastritis, reflux esophagitis, or gastrointestinal track damage caused by Helicobacter pylori. Further, the above compound as well as a pharmaceutically acceptable salt of the compound can also be used in the present invention.

Further, in addition to the compound represented by Chemical Formula 1, a pharmaceutically acceptable salt thereof can be used in the present invention. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively nontoxic and harmless to a patient and activates effectively and whose side effects do not degrade the beneficial efficacy of the above compound.

Pharmaceutically acceptable salts can be obtained by conventional methods using inorganic or organic acids. For example, the pharmaceutically acceptable salt can be prepared by dissolving the compound represented by Chemical Formula 1 in a water-miscible organic solvent, e.g., acetone, methanol, ethanol or acetonitrile, followed by adding an organic acid or an inorganic acid, and filtering and drying the precipitated crystals. Alternatively, it may be prepared by subjecting a solvent or an excessive amount of acid from the acid-added reaction mixture to reduced pressure and then drying the residue, or by adding a different organic solvent and then filtering the precipitated salt. At this time, the preferred salts may include salts derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxyrnaleic acid, benzoic acid, hydroxybenzoic acid, phenylacetic acid, cinnamic acid, salicylic acid, methanesulfonic acid, benzenesulfonic acid or toluenesulfonic acid, and the like.

In the present invention, the chemotherapeutic agent is used in combination with the compound represented by Chemical Formula 1 or a pharmaceutically acceptable salt thereof, and is used for the prevention or treatment of triple-negative breast cancer. Specifically, it refers to a substance that has cytotoxicity as a chemical substance for treating cancer. An example of the chemotherapeutic agent that can be used in the present invention include anthracyclines including Doxorubicin, and taxanes such as docetaxel and paclitaxel.

The disease to be prevented or treated in the present invention is triple-negative breast cancer, which is a breast cancer in which Estrogen Receptor (ER), Progesterone Receptor (PR), and Human Epidermal Growth Factor Receptor 2 (HER2) are all negative. Thus, therapeutic agents other than existing anticancer chemotherapy are limited, but by using the compound represented by Chemical Formula 1, or a pharmaceutically acceptable salt thereof, and a chemotherapeutic agent together as in the present invention, it is possible to prevent or treat triple-negative breast cancer. Further, the triple-negative breast cancer may be caused by V-ATPase overexpression.

As used herein, the term "prevention" refers to any act to delay or inhibit occurrence, spread or recurrence of the above-mentioned diseases by administration of the composition of the present invention, and "treatment" refers to any act to improve or change the symptoms of the above diseases for the better by administration of the composition of the present invention.

Preferably, the molar ratio of the compound represented by Chemical Formula 1, or a pharmaceutically acceptable salt thereof, and the chemotherapeutic agent is 5:1 or more. Also, preferably, the molar ratio of the compound represented by Chemical Formula 1, or a pharmaceutically acceptable salt thereof, and the chemotherapeutic agent is 30:1 or less.

More preferably, the molar ratio (a:b) of (a) the compound represented by Chemical Formula 1, or a pharmaceutically acceptable salt thereof, and (b) the chemotherapeutic agent is 6:1 or more, 7:1 or more, 8:1 or more, 9:1 or more, or 9.5:1 or more, and 25:1 or less, 24:1 or less, 23:1 or less, 22:1 or less, 21:1 or less, 20:1 or less, 19:1 or less, 18:1 or less, 17:1 or less, 16:1 or less, 15:1 or less, 14:1 or less, 13:1 or less, 12:1 or less, or 11:1 or less.

Further, when the chemotherapeutic agent is Doxorubicin, the molar ratio (a:b') of (a) the compound represented by Chemical Formula 1, or a pharmaceutically acceptable salt thereof, and (b') Doxorubicin is 9.5:1 or more and 11:1 or less.

Further, when the chemotherapeutic agent is docetaxel, the molar ratio(a:b") of (a) the compound represented by Chemical Formula 1, or a pharmaceutically acceptable salt thereof, and (b") docetaxel is preferably 1000:1 or more. More preferably, the molar ratio(a:b") of the compound represented by Chemical Formula 1, or a pharmaceutically acceptable salt thereof, and docetaxel is 100000:1 or less.

The pharmaceutical composition of the present invention can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present invention can be dissolved in oils, propylene glycol or other solvents commonly used in the preparation of injection solutions. Furthermore, the compounds of the present invention can be formulated in ointments or creams for topical application.

A preferred dose of the active ingredients included in the composition of the present invention (the compound represented by Chemical Formula 1 or a pharmaceutically acceptable salt thereof, and the chemotherapeutic agent) may be varied according to the condition and weight of a patient, the severity of a disease, the type of a drug, and the route and duration of administration, but it may be suitably selected by those skilled in the art. In order to achieve the desirable effects, however, each of the active ingredients included in the composition of the present invention may be administrated daily at a dose of 0.0001 to 100 mg/kg (body weight), and preferably 0.001 to 100 mg/kg (body weight). The administration may be performed once a day or in divided doses each day through an oral or parenteral route.

Depending on the method of administration, the pharmaceutical composition may contain the active ingredient in an amount of 0.001 to 99% by weight, preferably 0.01 to 60% by weight based on the total sum of the active ingredients.

The pharmaceutical composition according to the present invention may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

A pharmaceutical composition according to the present invention can be usefully used for preventing or treating triple-negative breast cancer.

### [BRIEF DESCRIPTION OF THE DRAWINGS]

FIG. 1 shows the results of Experimental Example 1 of the present invention, which is a graph showing the evaluation results of antitumor efficacy through combined administration of Doxorubicin.
FIG. 2 shows the results of Experimental Example 2 of the present invention, which is a graph showing the evaluation results according to the molar ratio for combination use with Doxorubicin.
FIG. 3 shows the results of Experimental Example 2 of the present invention, which is a graph showing the evaluation results according to the molar ratio for combination use with Esomeprazole.
FIG. 4 shows the results of Experimental Example 3 of the present invention, which is a graph showing the evaluation results of antitumor efficacy through combined administration of docetaxel.

### [DETAILED DESCRIPTION OF THE EMBODIMENTS]

Below, the present invention will be described in more detail to assist in the understanding of the invention. However, the following examples are provided for illustrative purposes only, and should not be construed as limiting the scope of the present invention to these examples.

### Preparation Example

The compound represented by Chemical Formula 1 of the present invention was prepared in the same manner as in Example 8 of Korean Patent Application No. 10-2016-0013588, which was hereinafter named "Fexuprazan".
Molecular weight: 446.87
¹H-NMR (500 MHz, MeOD): 7.69(s, 1H), 7.58-7.53(m, 1H), 7.45(t, 1H), 7.30(d, 1H), 7.20-7.15(m, 2H), 7.02-6.94(m, 2H), 4.07(d, 2H), 3.46(s, 3H), 2.71(s, 3H)

On the other hand, commercially available products of Doxorubicin and docetaxel were used as a chemotherapeutic agent for use with the above compound.

### Experimental Example 1: Evaluation of antitumor efficacy through combined administration of Doxorubicin

In order to confirm the anticancer efficacy when Fexuprazan and Doxorubicin were administered in combination, cell proliferation was measured for Hs578T (human triple-negative breast cancer cell line) by a CellTiter-Glo^{®} Luminescent Cell Viability (Promega, Catalog #G7571) method. The above evaluation was performed by a method in which when the luciferase enzyme was activated by ATP leaked from living cells, it reacted with a luciferin substrate, and the enzyme activity at this time was measured to confirm the cell viability.

Specifically, 100 µL of cell culture medium (RPMI medium, 10% FBS, 1% P/S, 4.5 g/L D-Glucose, 2.383 g/L HEPES Buffer, L-Glutamine, 1.5 g/L Sodium Bicarbonate, 110 mg/L Sodium pyruvate) was used and each cancer cell line was prepared in a white clear-bottom 96-well microplate. 10 µM of Fexuprazan and 1 µM of Doxorubicin were administered singly and in combination, respectively. As a comparative group, 10 µM of Vonoprazan was administered singly or in combination with Doxorubicin. At this time, 0.1% DMSO was used as a solvent for each composition. Further, 0.1% DMSO was used as a vehicle control. Each experimental group was repeated twice. Plates treated with the compound at respective concentrations were incubated in a 5% CO₂ incubator at 37 °C for 48 hours. 100 µL/well of the substrate aqueous solution containing luciferin was added to a white clear-bottom 96 well microplate to initiate the enzymatic reaction. The reaction was performed at room temperature in a dark room for 10 minutes, and luminescence was measured using a Flexstation3 multi-mode microplate reader. The luciferase enzyme activity, representing the amount of ATP, was measured by a chemiluminescence method according to CellTiter-Glo^{®} Luminescent Cell Viability instructions, and the inhibitory activity was calculated. The results of each compound were analyzed using Microsoft Excel. Compound concentrations were converted into logarithmic values using GraphPad Prism software and expressed on the graph.

**[Table 1]**

| Doxorubicin Conc. (µM) | Fexuprazan Conc. (µM) | Vonoprazan Conc. (µM) | Relative cell viability (%) |
|---|---|---|---|
| 0 | 0 | 0 | 100¹⁾ |
| 0 | 10.0 | 0 | 100 |
| 0 | 0 | 10.0 | 92.3 |
| 1.0 | 0 | 0 | 70.6 |
| 1.0 | 10.0 | 0 | 24.4 |
| 1.0 | 0 | 10.0 | 66.6 |
| 1) Vehicle control | | | |

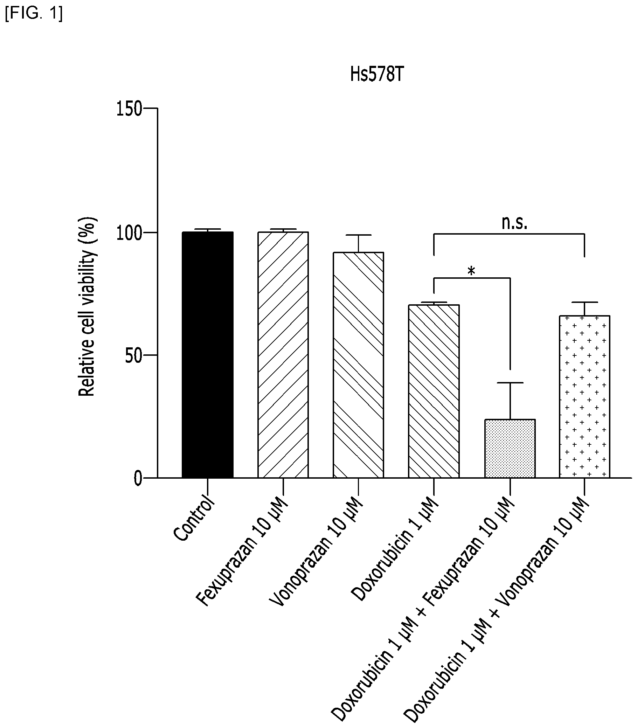

As can be seen in Table 1 and FIG. 1, it can be confirmed that Fexuprazan, when administered in combination with Doxorubicin as a chemotherapeutic agent, has a superior antitumor effect compared to when administered alone.

In addition, it can be confirmed that Vonoprazan has no synergistic effect with Doxorubicin, and from this, even in the case of a general gastric acid secretion inhibitor or P-CAB, it cannot be predicted whether a synergistic effect occurs when used together with Doxorubicin.

### Experimental Example 2: Confirmation of optimal molar ratio for combination use with Doxorubicin

In order to confirm the optimal combination ratio of Fexuprazan to Doxorubicin, cell proliferation was measured using a CellTiter-Glo^{®} Luminescent Cell Viability (Promega, Catalog #G7571) method in the same manner as in Experimental Example 1. In addition to a group in which 1 µM of Doxorubicin was administered alone to the Hs578T cell line, the experimental groups treated in combination with 1 µM of Doxorubicin and 0.1, 0.5, 1, 5, 10, 15, and 20 µM of Fexuprazan were compared, and 0.1% DMSO was used as a vehicle control. In addition, Esomeprazole was treated under the same conditions, and the combined effects of Esomeprazole and Fexuprazan were compared. The cells were incubated in a 5% CO₂ incubator at 37 °C for 48 hours, the luciferase enzyme activity, representing the amount of ATP, was measured by a chemiluminescence method according to CellTiter-Glo^{®} Luminescent Cell Viability instructions, and the inhibitory activity of the compound according to this purpose was calculated. The results of each compound were analyzed using Microsoft Excel, and significance was confirmed through an unpaired t test in Graphpad Prism software.

**[Table 2]**

| Doxorubicin Conc. (µM) | Fexuprazan Conc. (µM) | Esomeprazole Conc. (µM) | Relative cell viability (%) |
|---|---|---|---|
| 0 | 0 | 0 | 100¹⁾ |
| 1.0 | 0 | 0 | 50 |
| 1.0 | 0.1 | 0 | 61.2 |
| 1.0 | 0.5 | 0 | 54.8 |
| 1.0 | 1.0 | 0 | 61.5 |
| 1.0 | 5.0 | 0 | 40.8 |
| 1.0 | 10.0 | 0 | 20.7 |
| 1.0 | 15.0 | 0 | 3.7 |
| 1.0 | 20.0 | 0 | 1.0 |
| 1.0 | 0 | 0.1 | 58.2 |
| 1.0 | 0 | 0.5 | 58.6 |
| 1.0 | 0 | 1.0 | 56.9 |
| 1.0 | 0 | 5.0 | 52.8 |
| 1.0 | 0 | 10.0 | 48.4 |
| 1.0 | 0 | 15.0 | 37.6 |
| 1.0 | 0 | 20.0 | 40.7 |
| 1) Vehicle control | | | |

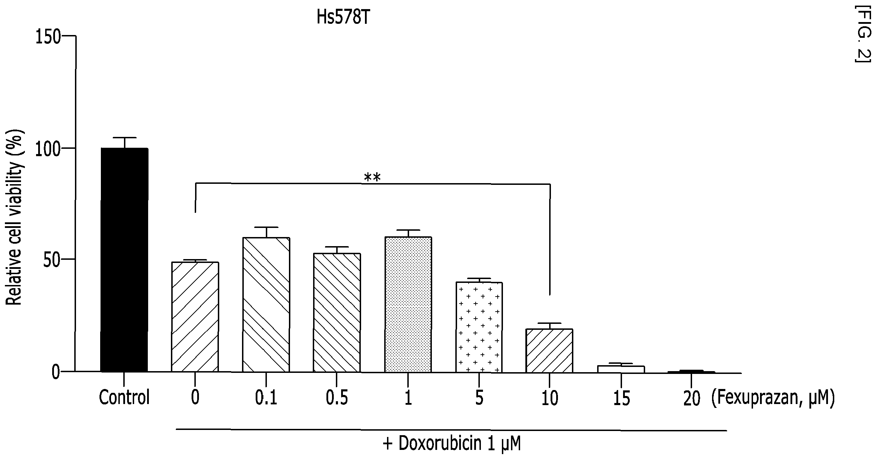

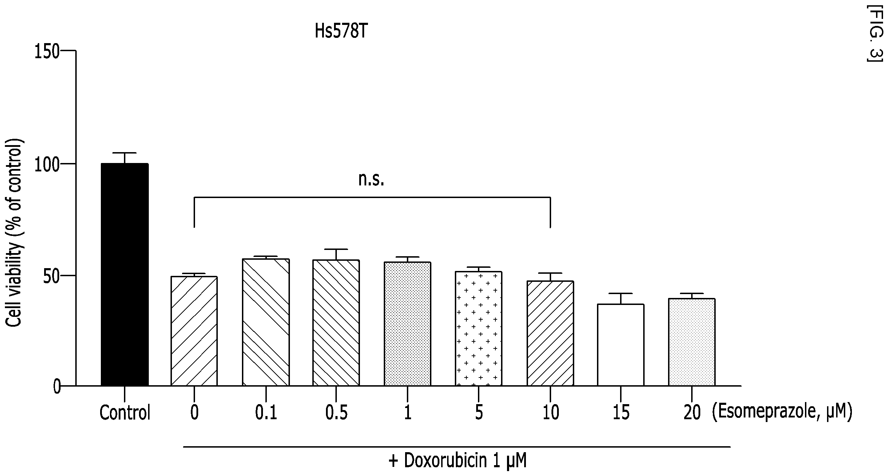

As can be seen in Table 2 and FIGS. 2 and 3, it was confirmed that as compared to the combined effect of Esomeprazole and chemotherapeutic agent, a synergistic effect shown when the molar ratio of Doxorubicin and Fexuprazan was 1:5 or more, it showed synergistic effects, and was especially superior at molar ratios of 1:10 or more.

### Experimental Example 3: Combined administration of docetaxel and evaluation of antitumor efficacy

In order to confirm the possibility of combined use of docetaxel and Fexuprazan, the degree of cell proliferation was measured when administered singly and in combination using a CellTiter-Glo^{®} Luminescent Cell Viability (Promega, Catalog #G7571) method in the same manner as in Experimental Example 1. Hs578T (human triple negative breast cancer cell line) was incubated in a 96 well clear bottom plate and then stabilized for 24 hours. 10 µM, which is the optimal concentration of Fexuprazan for combined use derived based on the previous experimental results, was used, and together with this, the concentration of docetaxel was made to be 1 nM and 10 nM, which are effective cytotoxic doses for cell viability, and treated with respective cells. As an experimental group for comparison, Esomeprazole was treated at 10 µM, the same as Fexuprazan, and 0.1% DMSO was used as vehicle control. After incubating in a 5% CO₂ incubator at 37 °C for 48 hours, the luciferase enzyme activity, representing the amount of ATP, was measured by a chemiluminescence method according to CellTiter-Glo^{®} Luminescent Cell Viability instructions, and the inhibitory activity was calculated. The respective results were analyzed using Microsoft Excel, and significance was confirmed through an unpaired t test in Graphpad Prism software.

**[Table 3]**

| Docetaxel Conc. (nM) | Fexuprazan Conc. (µM) | Esomeprazole Conc. (µM) | Relative cell viability (%) |
|---|---|---|---|
| 0 | 0 | 0 | 100¹⁾ |
| 1.0 | 0 | 0 | 88.3 |
| 1.0 | 10.0 | 0 | 78.4 |
| 1.0 | 0.0 | 10.0 | 91.5 |
| 10.0 | 0 | 0 | 49.3 |
| 10.0 | 10.0 | 0 | 43.3 |
| 10.0 | 0 | 10.0 | 61.1 |
| 1) Vehicle control | | | |

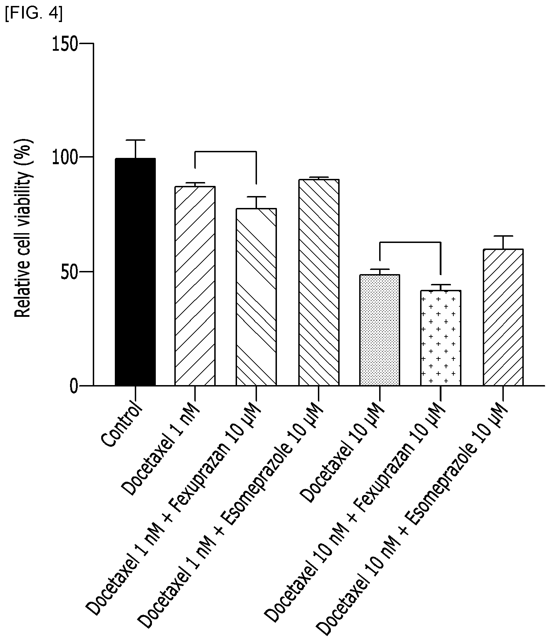

As can be seen in Table 3 and FIG. 4, it is confirmed that the chemotherapeutic agent docetaxel also has a synergistic effect on the antitumor effect relative to Esomeprazole.

## Claims

1. A pharmaceutical composition for preventing or treating triple-negative breast cancer comprising:
a compound represented by the following Chemical Formula 1, or a pharmaceutically acceptable salt thereof, and
a chemotherapeutic agent:

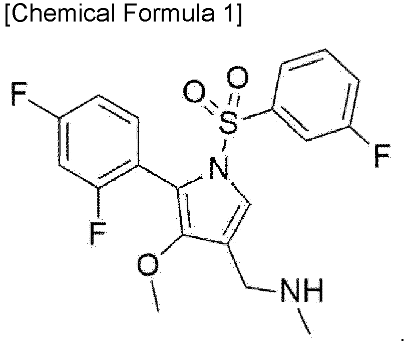

2. The pharmaceutical composition according to claim 1, wherein:
the chemotherapeutic agent is Doxorubicin, or docetaxel.

3. The pharmaceutical composition according to claim 1, wherein:
a molar ratio of the compound represented by Chemical Formula 1, or a pharmaceutically acceptable salt thereof, and the chemotherapeutic agent is 5:1 or more.

4. The pharmaceutical composition according to claim 1, wherein:
the chemotherapeutic agent is Doxorubicin, and
the molar ratio of the compound represented by Chemical Formula 1, or a pharmaceutically acceptable salt thereof, and Doxorubicin is 9.5:1 or more and 11:1 or less.

5. The pharmaceutical composition according to claim 1, wherein:
the chemotherapeutic agent is docetaxel, and
the molar ratio of the compound represented by Chemical Formula 1, or a pharmaceutically acceptable salt thereof, and docetaxel is 1000:1 or more.

6. The pharmaceutical composition according to claim 1, wherein:
the triple-negative breast cancer is caused by V-ATPase overexpression.
